# DEMANDE DE BREVET EUROPEEN

(11) **EP 2 105 165 A2**
(43) Date de publication de la demande: **30.09.2009**
(21) Numéro de dépôt: 09155258.8
(22) Date de dépôt: 16.03.2009
(51) Int. Cl.: A61Q 19/02, A61K 8/04, A61K 8/44, A61K 8/49, A61K 8/38

(54) **Composition cosmétique comprenant un dérivé d'acide imido-percarboxylique et un ester n-acylé d'acide aminé**

(30) Priorité: 28.03.2008 FR 0852018
(71) Demandeur: L'Oréal, 75008 Paris (FR)
(72) Inventeur: Provost, Roxane, 75013, Paris (FR); Remaut, Geoffroy, 94260, Fresnes (FR); Kermorvan, Cécile, 94100, Saint Maur des Fosses (FR); Moussay, Céline, 92170, Vanves (FR)
(74) Mandataire: Duvert, Sandra

(57) **Abrégé**

La présente invention se rapporte à une composition cosmétique ou dermatologique comprenant, dans un milieu physiologiquement acceptable, au moins un dérivé d'acide imido-percarboxylique et au moins un ester choisi parmi les esters d'acide aminés N-acylés de formule (A)

R'₁(CO)N(R'₂)CH(R'₃)(CH₂)ₙ(CO)OR'₄ (A)

dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

## Description

La présente invention se rapporte à une composition, notamment cosmétique ou dermatologique, comprenant un dérivé d'acide imido-percarboxylique et un ester d'acide aminé N-acylé.

Il est connu d'utiliser des dérivés d'acide imido-percarboxylique dans des compositions topiques, notamment cosmétiques ou dermatologiques, par exemple comme agents anti-microbien ou anti fongique. On connaît également leur activité dans des compositions anti acné ou blanchissant.
Cependant, l'utilisation de ces dérivés pose un problème dans la mesure où ces composés se présentent sous forme cristalline et où ils ne sont pas ou peu solubles dans l'eau et dans les corps gras traditionnellement utilisés dans les domaines cosmétique et dermatologique. Ainsi, introduits tels quels dans les compositions topiques, ils ne se solubilisent pas et restent à l'état de cristaux, ce qui peut entraîner des désagréments ou inconforts à l'utilisation de la peau.
Ces dérivés peuvent être partiellement solubilisés dans les alcools inférieurs comme l'éthanol ou l'isopropanol ou des solvants tels que l'octyldodécanol, certains glycols, les alcools gras à chaîne courte (inférieurs à C12). Cependant, les alcools inférieurs présentent l'inconvénient de dessécher et d'irriter la peau ; on préfère donc les utiliser en quantités réduites voire nulle dans les produits de soin du corps et/ou du visage. En outre, ces solubilisants ne peuvent être introduits qu'en de petites quantités sous peine d'altérer les qualités cosmétiques (douceur) et la stabilité des compositions les contenant.

Il subsiste donc le besoin de pouvoir solubiliser les dérivés d'acide imido-percarboxylique dans les milieux physiologiquement acceptables des compositions cosmétiques et/ou dermatologiques.

La demanderesse a trouvé de façon inattendue que des dérivés lipophiles d'aminoacides sous forme d'esters permettaient de solubiliser les dérivés d'acide imido-percarboxylique, sans que ne se produise une recristallisation de ces dérivés.

La présente invention a donc pour objet une composition comprenant dans un milieu physiologiquement acceptable, au moins un dérivé d'acide imido-percarboxylique et au moins un ester choisi parmi les esters d'acide aminés N-acylés de formule (A)

R'₁(CO)N(R'₂)CH(R'₃)(CH₂)ₙ(CO)OR'₄ (A)

dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

On entend par "milieu physiologiquement acceptable" un milieu compatible avec les matières kératiniques telles que la peau y compris le cuir chevelu, les muqueuses, les yeux et les cheveux.

Les inventeurs de la présente demande ont trouvé que l'utilisation de dérivés lipophiles d'aminoacides sous forme d'esters permettait d'augmenter de façon inattendue la solubilisation des dérivés d'acide imido-percarboxylique précédemment décrits et d'obtenir des compositions stables (pas de formation de cristaux), dans le temps, également stables lorsqu'on modifie la température et satisfaisantes quant aux propriétés cosmétiques.

Un autre objet de l'invention est constitué par un procédé de traitement cosmétique consistant à appliquer ladite composition sur les matières kératiniques.

L'invention a également pour objet un procédé de solubilisation d'un dérivé d'acide imido-percarboxylique par un ester d'acide aminés N-acylés de formule (A) telle que définie plus haut.

### Dérivé d'acide imido-percarboxyligue

Le dérivé d'acide imido-percarboxylique utilisé dans la composition selon l'invention est de préférence choisi parmi les composés de formule (I) qui suit. 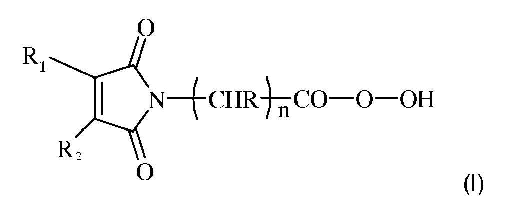 dans laquelle :
- R₁ et R₂ représentent indépendamment l'un de l'autre (i) un atome d'hydrogène, (ii) un groupe (C₁-C₅)alkyl éventuellement substitué par un ou plusieurs, de préférence par un à trois groupes choisis parmi un groupe (C₁-C₅)alcoxy, le groupe -OH, le groupe -COOH, le groupe -COOOH, un groupe -COOR₄, R₄, étant tel que défini ci-après, le groupe -NO₂ et un atome d'halogène ou (iii) un groupe (C₁-C₅)alcoxy,
- ou bien R₁ et R₂ forment ensemble
   - soit un cycle à 5 ou 6 chaînons non aromatique éventuellement substitué par un ou plusieurs, de préférence par un à trois groupes choisis parmi un groupe (C₁-C₅)alkyl éventuellement substitué par un ou plusieurs, de préférence par un à trois groupes choisis parmi un groupe (C₁-C₅)alcoxy, le groupe -OH, le groupe -COOH, le groupe -COOOH, un groupe - COOR₄, le groupe -NO₂ et un atome d'halogène ou un groupe (C₁-C₅)alcoxy et
- R et n sont tels que définis ci-après,
   - soit un cycle aromatique A pour former plus particulièrement un composé dérivé d'acide imido-aromatique percarboxylique de formule (II), 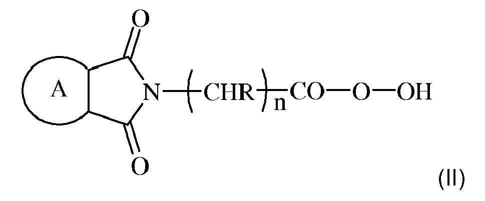
dans laquelle :
- A représente un cycle benzène ou naphtalène éventuellement substitué par un ou plusieurs, de préférence jusqu'à 4, et encore plus préférentiellement par 1 ou 2 groupes choisis parmi un radical R₃, un groupe (C₁-C₅)alcoxy, le groupe -OH, le groupe -COOH, le groupe -COOOH, un groupe -COOR₄, le groupe -NO₂ et un atome d'halogène,
- le ou les groupes R, identiques ou différents, représent(ent) un atome d'hydrogène, un groupe -OH, un groupe -COOH, un groupe -COOOH, un groupe
- COOR₄ ou un radical R₃,
   ○ R₃ représente un groupe (C₁-C₅)alkyle éventuellement substitué par un ou plusieurs, de préférence par un à trois groupes choisis parmi un groupe (C₁-C₅)alcoxy, le groupe -OH, le groupe -COOH, le groupe - COOOH, un groupe -COOR₄, le groupe -NO₂ et un atome d'halogène,
   ○ R₄ représente un groupe (C₁-C₅)alkyle éventuellement substitué par un groupe choisi parmi un groupe (C₁-C₅)alcoxy, le groupe -OH, le groupe - COOH, le groupe -COOOH, et un groupe -COOR₅ où R₅ est un groupe (C₁-C₅)alkyle, et
- n est un entier variant de 1 à 5, par exemple de 1 à 3 ou par exemple de 3 à 5.

Il est entendu que le composé de formule (I) ou (II) comprend tous les énantiomères éventuels et leurs mélanges ainsi que les sels correspondants ou tout dimère du composé de formule (I) ou (II).

Parmi les sels, on peut citer les sels de potassium ou les sels d'ammonium par exemple.

Des exemples de groupes (C₁-C₅)alkyle sont le méthyle, l'éthyle, n- et i-propyle, n-, i-, sec- et ter-butyle et le pentyle. Parmi les groupes (C₁-C₅)alcoxy, on peut citer le méthoxy ou l'éthoxy. Parmi les atomes d'halogène, on peut citer le fluor, le chlore et le brome.
Parmi les groupes (C₁-C₅)alkyle substitués on cite notamment les groupes -CH₂OCH₃, -CH₂OC₂H₅, -CH₂OH, -CH₂COOH, -CH₂COOOH, -CH₂CH₂COOH, -CH₂CH₂ COOOH, -CH₂Cl, -CH₂F, -CF₃ et -CH₂COOC₂H₅.
Selon un mode de réalisation, le groupe R est un atome d'hydrogène.
Selon un autre mode de réalisation, A représente un cycle benzène.
Selon encore un autre mode de réalisation, A est substitué par au moins un groupe -COOH ou un groupe -COOOH.
Selon enfin un autre mode de réalisation, A n'est pas substitué.

Selon un mode de réalisation, on utilise un dérivé d'acide imido-aromatique percarboxylique de formule (II) pour laquelle A est un cycle benzène ou naphtalène non substitué, R est un atome d'hydrogène, un groupe -COOOH ou un groupe (C₁-C₃)alkyle éventuellement substitué par un groupe -COOOH ou un groupe -COOH, et n varie de 3 à 5.

Parmi les composés de formule (I), on peut citer les composés suivants : l'acide 2,5-dihydro-3-méthyl-2,5-dioxo-1H-pyrrole-1-hexanepéroxoïque (R₁=H, R₂=Me ; n=5) ; l'acide 2,5-dihydro-2,5-dioxo-1H-pyrrole-1-hexanepéroxoïque (R₁=R₂=H ; n=5), et l'acide 1,3,4,5,6,7-hexahydro-1,3-dioxo-2H-isoindole-2-butanepéroxoïque (R₁ et R₂ = cycle à 6 chaînons ; n=3).

Parmi les composés de formule (II), on peut citer, en noms usuels, les composés suivants : l'acide phthalimido-péracétique (A=benzène; n=1), l'acide 3-phthalimido-perpropionique (A=benzène ; n=2), l'acide 2-phthalimido-di-persuccinique (A=benzène ; R=COOOH ; n=2), l'acide 3-phthalimido-perbutyrique (A=benzène ; R=CH₃ ; n=2), l'acide 2-phthalimido-per-propionique (A=benzène ; R=CH₃ ; n=1), l'acide méthyle demi-ester de 2-phthalimido-mono-per-glutarique (A=benzène ; R= -COOOMe ; n=3), l'acide 3-phthalimido-diperadipique (A=benzène ; R=COOOH; n=4), l'acide naphthalimido-peracétique (A=napthalène ; n=1), l'acide 2-phthalimido-mono-persuccinique (A=benzène ; R=COOH ; n=2) et l'acide 4-(4-percarboxy)-phthalimido-per-butyrique.
On peut encore citer, parmi les composés de formules (II), les composés suivants, cités en noms CAS :
- des composés pour lesquels A est un cycle benzène, par exemple l'acide 1,3-dihydro-1,3-dioxo-2H-isoindole-2-butanepéroxoïque, encore nommé acide phthalimidopéroxybutanoïque, l'acide 3-phthalimidopéroxybutanoïque ou acide 4-phthalimidoperbutyrique (n=3), l'acide 1,3-dihydro-1,3-dioxo-2H-isoindole-2-pentanepéroxoïque (n=4), l'acide 1,3-dihydro-1,3-dioxo-2H-isoindole-2-heptanepéroxoïque (n=6), l'acide 1,3-dihydro-1,3-dioxo-2H-Isoindole-2-octanepéroxoïque (n=7) ou acide phthalimidopéroctanoïque, l'acide 1,3-dihydro-1,3-dioxo 2H-isoindole-2-nonanepéroxoïque (n=8), l'acide 1,3-dihydro-1,3-dioxo-2H-isoindole-2-decanepéroxoïque (n=9), l'acide 1,3-dihydro-1,3-dioxo-2H-isoindole-2-undecanepéroxoïque (n=10), l'acide 1,3-dihydro-1,3-dioxo-2H-isoindole-2-dodecanepéroxoïque (n=11) ;
- des composés pour lesquels A est un cycle benzène substitué, par exemple l'acide 1,3-dihydro-5-(hydropéroxycarbonyl)-1,3-dioxo-2H-isoindole-2-butanepéroxoïque (n=3, substitution par un groupe -COOOH), l'acide 1,3-dihydro-4-(hydropéroxycarbonyl)-1,3-dioxo-2H-isoindole-2-butanepéroxoïque (n=3, substitution par un groupe -COOOH) ;
- des composés pour lesquels A est un cycle benzène et au moins un des groupes R est différent d'un atome d'hydrogène, par exemple l'acide 3-(1,3-dihydro-1,3-dioxo-2H-isoindol-2-yl)-hexanedipéroxoïque (n=3, R=CH₂COOOH), l'acide (1,3-dihydro-1,3-dioxo-2H-isoindol-2-yl)-butanedipéroxoïque (R=COOOH, n = 2), l'acide 2-(1,3-dihydro-1,3-dioxo-2H-isoindol-2-yl)-pentanedipéroxoïque (R=COOOH, n = 3), l'ester 5-méthyl-glutarique d'acide 4-phthalimido-monopéroxyacide, (R=COOMe, n = 3), l'acide 1,3-dihydro-β-méthyl-1,3-dioxo-2H-isoindole-2-propanepéroxoïque encore nommé acide β-méthyl-1,3-dioxo-péroxy-2-isoindolinepropionique ou acide 3-phthalimidoperbutyrique (R = Me, n = 2).
Le sel suivant peut également être nommé : sel 1-dodecanaminium, N-(2-hydroxyethyl)-N,N-diméthyl d'acide 1,3-dihydro-1,3-dioxo-2H-isoindole-2-hexaneperoxoïque (1:1).

Selon encore un autre mode, le composé de formule (II) est l'acide phthalimidopéroxycaproïque, encore nommé acide 6-(phthalamidopéroxy)hexanoïque, acide ε-(phtalimidopéroxy)hexanoïque ou encore PAP. Sa structure chimique est la suivante : 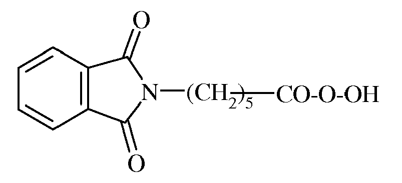 Il correspond à un composé de formule (II) dans laquelle le groupe R représente un atome d'hydrogène, A représente un cycle benzène non substitué et n=5.

Enfin, à titre de dimère acceptable d'un composé de formule (II), on peut citer le dimère du PAP encore nommé acide 5,7-dihydro-1,3,5,7-tetraoxo-benzo[1,2-c:4,5-c']dipyrrole-2,6(1H,3H)-dihexanepéroxoïque de formule

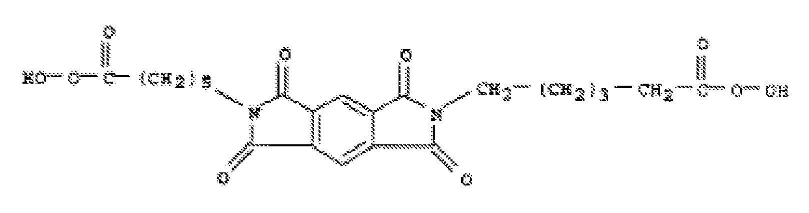

A titre d'exemple, la quantité en matières actives de dérivé d'acide imido-percarboxylique, en particulier de composé de formule (I) ou (II), utilisable selon l'invention peut aller par exemple de 0,01 à 5 % en poids, notamment de 0,01 à 2 % en poids, de préférence de 0,15 à 1,5% en poids et mieux de 0,3 à 1 % en poids, par rapport au poids total de la composition.

L'acide phthalamidopéroxycaproïque ou phthalamidopéroxyhexanoïque (PAP) est notamment disponible sous deux formes commerciales par la société SOLVAY.
Ainsi, l'acide phthalamidopéroxycaproïque peut être mis en oeuvre conjointement avec des cyclodextrines, de sorte à améliorer la stabilité du composé.
Le document EP 895 777, déjà cité en préambule, décrit plus particulièrement la formation d'un adduit d'acide ε-phthalamidopéroxyhéxanoïque avec la cyclodextrine.
Un tel produit encapsulé est vendu par la société SOLVAY sous le nom commercial. EURECO^{®}HC-P11 (Powdered inclusion complex of Phthalimidoperoxycaproicacid with pharmagrade β-cyclodextrin).

L'acide phthalamidopéroxycaproïque est également fourni sous la forme d'une dispersion aqueuse (à 17 % en matière active) sous le nom commercial EURECO^{®} HC L17.
L'acide phthalamidopéroxycaproïque est également fourni sous la forme d'une poudre sous le nom commercial EURECO^{®}HC-P11.
De telles dispersions aqueuses d'acide phthalamidopéroxycaproïque sont décrites dans le document EP 1 074 607. Ces dispersions aqueuses sont en réalité stabilisées par des copolymères d'éther méthylvinylique avec l'acide et/ou l'anhydride maléique, dans un rapport 1:1, ayant une structure alternée.

D'autres formes galéniques comprenant un dérivé d'acide imido-percarboxylique peuvent également convenir à la mise en oeuvre de la présente invention. A ce titre, on peut notamment citer les dispersions notamment aqueuses, telles que décrites dans le demande de brevet WO 2004/110610, les capsules comprenant une matrice à base de gel telles que décrites dans la demande de brevet WO 2004/110611, les capsules à base d'un sel inorganique telles que décrites dans la demande WO 2004/110612 ou encore les capsules multicouches comprenant au moins un polyélectrolyte et/ou un tensioactif ionique telles que décrites dans la demande de brevet WO 2004/110613.

### Esters d'acide aminés N-acylés

L'ester présent dans la composition selon l'invention est avantageusement choisi parmi les esters d'acide aminés N-acylés de formule

R'₁(CO)N(R'₂)CH(R'₃)(CH₂)ₙ(CO)OR'₄

dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

Dans la formule des esters d'aminoacide présentées ci-dessus, le groupement R'₁(CO)- est un groupement acyle d'un acide choisi de préférence dans le groupe formé par l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste. Ces acides gras peuvent en outre présenter un groupe hydroxyle. De manière encore plus préférée, il s'agira de l'acide laurique.

La partie -N(R'₂)CH(R'₃)(CH₂)ₙ(CO)- de l'ester d'aminoacide est de préférence choisie parmi les aminoacides suivants : glycine, alanine, valine, leucine, isoleucine, sérine, thréonine, proline, hydroxyproline, β-alanine, acide aminobutyrique, acide aminoca-proique, sarcosine, ou N-méthyl-β-alanine.
De manière encore plus préférée, il s'agira de la sarcosine.
La partie des esters aminoacides correspondant au groupe OR'₄ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, huile de fusel, pentanol, héxanol, cyclohéxanol, octanol, 2-éthylhéxanol, décanol, alcool laurylique, alcool myristique, alcool cétylique, alcool cétostéarylique, alcool stéarylique, alcool oléique, alcool béhénylique, alcool de jojoba, alcool 2-héxadécylique, alcool 2-octyldodécanol et alcool isostéarylique.
Ces esters d'acide aminé peuvent en particulier être obtenus à partir de sources naturelles en acides aminés. Dans ce cas, les acides aminés proviennent d'hydrolyse de protéines naturelles végétales (avoine, blé, soja, palme, coco) et conduisent alors nécessairement à des mélanges d'acides aminés qui devront ensuite être estérifiés puis N-acylés. La préparation de tels acides aminés est plus particulièrement décrite dans la demande de brevet FR 2 796 550 qui est incorporée ici par référence.

L'ester d'acide N-acylé aminé plus particulièrement préféré pour son utilisation dans la présente invention est le N-lauroylsarcosinate d'isopropyl de formule : 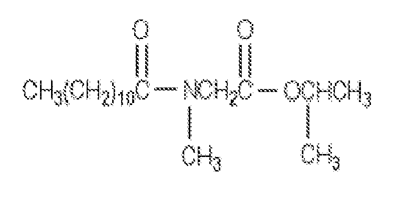 tel que le produit vendu sous le nom ELDEW SL-205 par la société Ajimoto.

L'ester d'acide N-acylé aminé tel que décrit ci-dessus peut être présent dans la composition selon l'invention en une teneur allant de 0,05 à 50% en poids, de préférence de 1 à 30% en poids et plus préférentiellement de 1 à 10% en poids par rapport au poids total de la composition.

Selon un mode de réalisation préféré, l'ester d'acide aminé N-acylé et le dérivé d'acide imido-percarboxylique de formule (I) sont présents dans la composition selon l'invention en des teneurs en matières actives telles que le ratio ester d'acide aminé N-acylé sur dérivé d'acide imido-percarboxylique allant de 5 à 25, et mieux de 10 à 20.

La composition peut également comprendre une phase grasse, qui peut comprendre des huiles, des gommes, des cires usuellement utilisées dans le domaine d'application considéré. Comme huiles ou cires utilisables dans l'invention, on peut citer les huiles minérales (huile de vaseline), les huiles végétales (fraction liquide du beurre de karité, huile de tournesol, huile d'abricot, de son de riz...), les huiles animales (perhydrosqualène), les huiles de synthèse (huile de Purcellin), les huiles ou cires siliconées (cyclométhicone, dimethicone) et les huiles fluorées (perfluoropolyéthers), les cires d'abeille, de carnauba, ou paraffine, le beurre de karité, l'huile de jojoba hydrogénée. On peut ajouter à ces huiles des alcools gras (cétyliques, stéaryliques, ...) et des acides gras (acide stéarique, ....).

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La composition peut contenir également des adjuvants habituels dans le domaine considéré, tels que les tensioactifs, les émulsionnants, les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes, d'autres actifs cosmétiques ou pharmaceutiques. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme tensioactifs susceptibles d'être utilisés, on peut citer par exemple le stéarate de glycérol, le polysorbate 60 et le mélange de PEG-6/PEG-32/Glycol Stéarate vendu sous la dénomination de TefoseR 63 par la société Gattefosse ; des dérivés PEG stéarate, des dérivés de sucres.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut se présenter sous toutes les formes galéniques envisageables.
Notamment, la composition peut avoir la forme d'une solution aqueuse, alcoolique, hydroalcoolique ou huileuse; d'une dispersion du type lotion ou sérum; d'une émulsion eau-dans-huile, huile-dans-eau ou multiple; d'une suspension; de microcapsules ou microparticules; de dispersions vésiculaires de type ionique et/ou non ionique; d'une lotion aqueuse, huileuse ou sous forme de sérum; d'une mousse, d'une préparation solide par exemple de stick; d'une composition pour aérosol comprenant également un agent propulseur sous pression ou sous forme d'un patch.
La composition utilisable selon l'invention peut se présenter sous la forme d'une composition pour soins capillaires, notamment un shampooing, une lotion de mise en plis, une lotion traitante, une crème ou un gel coiffant, une composition de teinture, notamment d'oxydation, des lotions restructurantes pour les cheveux, une composition de permanente (notamment une composition pour le premier temps d'une permanente), une lotion ou un gel antichute, un shampooing antiparasitaire.
Elle peut également se présenter sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, comme des lotion de nettoyage, composition de bronzage artificiel); une composition de maquillage du corps ou du visage telle qu'un fond de teint; une composition pour le bain; une composition désodorisante comprenant par exemple un agent bactéricide; une composition après-rasage; une composition épilatoire; une composition contre les piqûres d'insectes; une composition anti-douleur; une composition pour traiter certaines maladies de la peau comme l'eczéma, la rosacée, le psoriasis, les lichens, les prurits sévères.
Lorsque la composition utilisable selon l'invention est destinée à un usage de type peeling, elle peut également se présenter sous toutes les formes galéniques évoquées ci-dessus pour autant qu'elle s'élimine facilement par rinçage, et notamment sous forme de gel aqueux, de solution aqueuse ou hydroalcoolique. Elle peut être appliquée par tout moyen permettant une répartition uniforme et notamment à l'aide d'un coton, d'une tige, d'un pinceau, d'une gaze, d'une spatule ou d'un tampon, ou encore par pulvérisation, et peut être éliminée par rinçage à l'eau ou à l'aide d'un détergent doux. Selon un mode préféré de réalisation de l'invention, la composition destinée au peeling chimique renferme une phase aqueuse continue.
Elle peut se présenter sous toutes les formes normalement utilisées pour une application topique, notamment sous forme d'une solution hydroalcoolique, d'une émulsion huile-dans-eau ou eau-dans-huile ou multiple, d'un gel huileux, ou d'un produit anhydre liquide, pâteux ou solide ou sous forme de dispersion en présence de sphérules, ces sphérules peuvent être des nanoparticules polymériques telles que des nanosphères ou des nanocapsules ou des vésicules lipidiques de type ionique ou non-ionique. Ces compositions sont préparées selon les méthodes usuelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou les cheveux sous forme d'aérosol. Elle peut également se présenter sous forme solide, et par exemple sous forme de stick.

Les exemples suivants illustrent de manière non limitative la présente invention. Sauf indication contraire, les quantités indiquées sont exprimées en pourcentage massique.

### Exemple 1 : gel aqueux

| | % en poids |
|---|---|
| Hydroxyde de sodium | 0,37 |
| Acide citrique | 0,37 |
| Acide phthalimidopéroxycaproïque | |
| Eureco^{®} HCL17 (17 % en poids en matière active) de SOLVAY | 5 |
| Alcool cétylique | 1 |
| N-lauroylsarcosinate d'isopropyle (ELDEW SL-205 d'Ajimoto) | 10 |
| Mélange stéarate de glycéryle/stéarate de polyéthylène glycol (100 OE) | 0,6 |
| (ARLACEL 165FL de Croda) | |
| Polymère réticulé acrylates/acrylate d'alkyl en C10-C30 (Carbopol | 0,9 |
| Ultrez-20 de Noveon) | |
| Polyéthylène glycol (8 OE) | 6 |
| Conservateur | 0,2 |
| Eau | Qsp100 |

### Mode opératoire

Le Carbopol est saupoudré dans la phase aqueuse à température ambiante (25°C) et l'on attend 10 minutes que la poudre s'imprègne d'eau. L'ensemble est placé sous agitation au Rayneri puis on ajoute la soude en augmentant l'agitation. Après obtention d'un gel sans grumeaux, on ajoute les conservateurs, l'acide citrique et le PEG-8, puis le PAP (préalablement homogénéisé quelques minutes au barreau). On maintient l'agitation élevée quelques minutes. Parallèlement on prépare la phase grasse en chauffant à 50°C l'alcool cétylique, le N-lauroylsarcosinate et l'Arlacel puis on émulsionne la phase grasse dans le gel aqueux précédemment préparé pendant 10 minutes sous forte agitation.

### Exemples 2 à 7 comparatifs

On a préparé les compositions suivantes :

| | Exemple 2 invention % en poids | Exemple 3 comparatif % en poids | Exemple 4 comparatif % en poids | Exemple 5 comparatif % en poids | Exemple 6 comparatif % en poids | Exemple 7 comparatif % en poids |
|---|---|---|---|---|---|---|
| **Phase A** | | | | | | |
| Hydroxyde de sodium | 0,2 | 0,2 | 0,2 | 0,2 | 0,2 | 0,2 |
| Disodium EDTA | 0,05 | 0,05 | 0,05 | 0,05 | 0,05 | 0,05 |
| Citrate trisodique | 0,05 | 0,05 | 0,05 | 0,05 | 0,05 | 0,05 |
| Gluconate de zinc | 0,1 | 0,1 | 0,1 | 0,1 | 0,1 | 0,1 |
| Acide citrique | 0,3 | 0,3 | 0,3 | 0,3 | 0,3 | 0,3 |
| Acide salicylique | 0,6 | 0,6 | 0,6 | 0,6 | 0,6 | 0,6 |
| Extrait de feuilles | | | | | | |
| | 0,02 | 0,02 | 0,02 | 0,02 | 0,02 | 0,02 |
| d'Eucalyptus | | | | | | |
| Gomme de xanthane | 0,2 | 0,2 | 0,2 | 0,2 | 0,2 | 0,2 |
| Glycérol | 5 | 5 | 5 | 5 | 5 | 5 |
| Conservateurs | 1 | 1 | 1 | 1 | 1 | 1 |
| Eau | Qsp100 | Qsp100 | Qsp100 | Qsp100 | Qsp100 | Qsp100 |

| **Phase B** | | | | | | |
|---|---|---|---|---|---|---|
| Octyl-2 dodécanol | 1 | 1 | 1 | 1 | 1 | 1 |
| acide n-octanoyl 5- | | | | | | |
| | 0,1 | 0,1 | 0,1 | 0,1 | 0,1 | 0,1 |
| salicylique | | | | | | |
| Polydimethylsiloxane, | | | | | | |
| 100 cSt (DC 200 Fluid | 0,5 | 0,5 | 0,5 | 0,5 | 0,5 | 0,5 |
| de de Dow Corning) | | | | | | |
| Cyclohexadiméthylsilox | | | | | | |
| ane, 8 cSt (Dc 246 Fluid | 8,5 | 8,5 | 8,5 | 8,5 | 8,5 | 8,5 |
| de Dow Corning) | | | | | | |
| Alcool cétylique | 0,25 | 0,25 | 0,25 | 0,25 | 0,25 | 0,25 |
| **N-lauroylsarcosinate** | | | | | | |
| **d'isopropyle (ELDEW** | **7,5** | | | | | |
| **SL-205 d'Ajimoto)** | | | | | | |
| **Carbonate de** | | **7,5** | | | | |
| **propylène** | | | | | | |
| **Hexylecanol** | | | **7,5** | | | |
| **Octyl-2 dodécanol** | | | | **7** | | |
| **Benzoates d'alcool en** | | | | | **7** | |
| **C12-C13 ramifiés** | | | | | | |
| **(COSMACOL ELI de** | | | | | | |
| **Sasol)** | | | | | | |
| **Polyéthylène glycol** | | | | | | **7,5** |
| **400 (8 OE)** | | | | | | |
| Mélange stéarate de | | | | | | |
| glycéryle/stéarate de | | | | | | |
| polyéthylène glycol | 1 | 1 | 1 | 1 | 1 | 1 |
| (100 OE) (ARLACEL | | | | | | |
| 165FL de Croda) | | | | | | |

| **Phase C** | | | | | | |
|---|---|---|---|---|---|---|
| Acide poly | | | | | | |
| acrylamidométhyl | | | | | | |
| propane sulfonique | | | | | | |
| réticulé et partiellement | | | | | | |
| | 2 | 2 | 2 | 2 | 2 | 2 |
| neutralisé à | | | | | | |
| l'ammoniaque | | | | | | |
| (Hostarecin AMPS de | | | | | | |
| Clariant) | | | | | | |

| **Phase D** | | | | | | |
|---|---|---|---|---|---|---|
| Acide | | | | | | |
| phthal imidopéroxycapro | | | | | | |
| ïque | 2,5 | 2,5 | 2,5 | 2,5 | 2,5 | 2,5 |
| Eureco^{®} HCL17 (17% | | | | | | |
| en poids en matière | | | | | | |
| active) de SOLVAY | | | | | | |

| **Phase E** | | | | | | |
|---|---|---|---|---|---|---|
| Alcool éthylique | 5 | 5 | 5 | 5 | 5 | 5 |

| **Phase F** | | | | | | |
|---|---|---|---|---|---|---|
| Fibres de polyamide 0,9 | | | | | | |
| DTex, long. 0,3 mm | 3 | 3 | 3 | 3 | 3 | 3 |
| (Utexbel) | | | | | | |

### Mode opératoire

On chauffe la phase A avec une partie de l'eau à 80°C sous agitation au Rayneri puis on refroidit à 65°C puis on ajoute la phase B, préalablement chauffée au bain-marie, dans la phase A sous agitation au mixeur (Phase de mise en émulsion). On dilue le mélange ramené à 45°C avec le reste de l'eau puis on ajoute successivement les phases C, D, E et F sous agitation. La phase D peut également être rajoutée pendant l'étape de dilution, avant ajout de la phase C.

On a ensuite évalué l'aspect des compositions :

| | **Exemple 2 invention** | **Exemple 3 comparatif** | **Exemple 4 comparatif** | **Exemple 5 comparatif** | **Exemple 6 comparatif** |
|---|---|---|---|---|---|
| **Aspect** | émulsion homogène, pas de cristaux | émulsion grossière | présence de cristaux, globules graisseux apparents | émulsion très grossière, présence de cristaux | émulsion grossière, présence de cristaux |

On constate que contrairement aux autres solvants, seul l'ester d'amino acide N-acylé selon l'invention permet une bonne solubilisation de l'acide phthalimidopéroxycaproïque et l'obtention d'une émulsion homogène, sans cristaux.

## Revendications

1. Composition cosmétique ou dermatologique comprenant, dans un milieu physiologiquement acceptable, au moins un dérivé d'acide imido-percarboxylique et au moins un ester choisi parmi les esters d'acide aminé N-acylé de formule (A)
R'₁(CO)N(R'₂)CH(R'₃)(CH₂)ₙ(CO)OR'₄ (A)
dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.

2. Composition selon la revendication 1, dans laquelle le dérivé d'acide imido-percarboxylique est un dérivé de formule (I) : 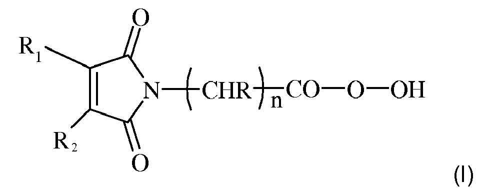 dans laquelle :
- R₁ et R₂ représentent indépendamment l'un de l'autre (i) un atome d'hydrogène, (ii) un groupe (C₁-C₅)alkyl éventuellement substitué par un ou plusieurs, de préférence par un à trois groupes choisis parmi un groupe (C₁-C₅)alcoxy, le groupe -OH, le groupe -COOH, le groupe -COOOH, un groupe -COOR₄, R₄, étant tel que défini ci-après, le groupe -NO₂ et un atome d'halogène ou (iii) un groupe (C₁-C₅)alcoxy,
- ou bien R₁ et R₂ forment ensemble
• soit un cycle à 5 ou 6 chaînons non aromatique éventuellement substitué par un ou plusieurs, de préférence par un à trois groupes choisis parmi un groupe (C₁-C₅)alkyl éventuellement substitué par un ou plusieurs, de préférence par un à trois groupes choisis parmi un groupe (C₁-C₅)alcoxy, le groupe -OH, le groupe -COOH, le groupe -COOOH, un groupe - COOR₄, le groupe -NO₂ et un atome d'halogène ou un groupe (C₁-C₅)alcoxy et
- R et n sont tels que définis ci-après,
• soit un cycle aromatique A pour former plus particulièrement un composé dérivé d'acide imido-aromatique percarboxylique de formule (II), 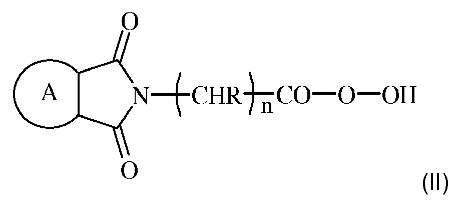
dans laquelle :
- A représente un cycle benzène ou naphtalène éventuellement substitué par un ou plusieurs, de préférence jusqu'à 4, et encore plus préférentiellement par 1 ou 2 groupes choisis parmi un radical R₃, un groupe (C₁-C₅)alcoxy, le groupe -OH, le groupe -COOH, le groupe -COOOH, un groupe -COOR₄, le groupe -NO₂ et un atome d'halogène,
- le ou les groupes R, identiques ou différents, représent(ent) un atome d'hydrogène, un groupe -OH, un groupe -COOH, un groupe -COOOH, un groupe -COOR₄ ou un radical R₃,
o R₃ représente un groupe (C₁-C₅)alkyle éventuellement substitué par un ou plusieurs, de préférence par un à trois groupes choisis parmi un groupe (C₁-C₅)alcoxy, le groupe -OH, le groupe -COOH, le groupe - COOOH, un groupe -COOR₄, le groupe -NO₂ et un atome d'halogène,
o R₄ représente un groupe (C₁-C₅)alkyle éventuellement substitué par un groupe choisi parmi un groupe (C₁-C₅)alcoxy, le groupe -OH, le groupe - COOH, le groupe -COOOH, et un groupe -COOR₅ où R₅ est un groupe (C₁-C₅)alkyle, et
- n est un entier variant de 1 à 5, par exemple de 1 à 3 ou par exemple de 3 à 5.

3. Composition selon la revendication 2, dans laquelle R est un atome d'hydrogène.

4. Composition selon l'une des revendications 2 ou 3, dans laquelle A est un cycle benzène.

5. Composition selon l'une quelconque des revendications 1 à 4, dans laquelle le composé de formule (I) est choisi parmi l'acide 2,5-dihydro-3-méthyl-2,5-dioxo-1H-pyrrole-1-hexanepéroxoïque, l'acide 2,5-dihydro-2,5-dioxo-1H-pyrrole-1-hexanepéroxoïque, et l'acide 1,3,4,5,6,7-hexahydro-1,3-dioxo-2H-isoindole-2-butaneperoxoïque.

6. Composition selon l'une quelconque des revendications précédentes, dans laquelle le dérivé d'acide imido-percarboxylique est choisi parmi l'acide phthalimido-péracétique, l'acide 3-phthalimido-perpropionique, l'acide 2-phthalimido-di-persuccinique, l'acide 3-phthalimido-perbutyrique, l'acide 2-phthalimido-per-propionique, l'acide méthyle demi-ester de 2-phthalimido-mono-per-glutarique, l'acide 3-phthalimido-diperadipique, l'acide naphthalimido-peracétique, l'acide 2-phthalimido-mono-persuccinique, l'acide 4-(4-percarboxy)-phthalimido-per-butyrique, l'acide 1,3-dihydro-1,3-dioxo-2H-isoindole-2-butanepéroxoïque, l'acide 3-phthalimidopéroxybutanoïque, l'acide 1,3-dihydro-1,3-dioxo-2H-isoindole-2-pentanepéroxoïque, l'acide 1,3-dihydro-1,3-dioxo-2H-isoindole-2-heptanepéroxoïque, l'acide 1,3-dihydro-1,3-dioxo-2H-Isoindole-2-octanepéroxoïque, l'acide 1,3-dihydro-1,3-dioxo-2H-isoindole-2-nonanepéroxoïque, l'acide 1,3-dihydro-1,3-dioxo-2H-isoindole-2-decanepéroxoïque, l'acide 1,3-dihydro-1,3-dioxo-2H-isoindole-2-undecanepéroxoïque, l'acide 1,3-dihydro-1,3-dioxo-2H-isoindole-2-dodecanepéroxoïque, l'acide 1,3-dihydro-5-(hydropéroxycarbonyl)-1,3-dioxo-2H-isoindole-2-butanepéroxoïque, l'acide 1,3-dihydro-4-(hydropéroxycarbonyl)-1,3-dioxo-2H-isoindole-2-butanepéroxoïque, l'acide 3-(1,3-dihydro-1,3-dioxo-2H-isoindol-2-yl)-hexanedipéroxoïque, l'acide (1,3-dihydro-1,3-dioxo-2H-isoindol-2-yl)-butanedipéroxoïcque, l'acide 2-(1,3-dihydro-1,3-dioxo-2H-isoindol-2-yl)-pentanedipéroxoïque, l'ester 5-méthyl-glutarique d'acide 4-phthalimido-monopéroxyacide, l'acide 1,3-dihydro-β-méthyl-1,3-dioxo-2H-isoindole-2-propanepéroxoïque et le sel 1-dodecanaminium, N-(2-hydroxyethyl)-N,N-diméthyl d'acide 1,3-dihydro-1,3-dioxo-2H-isoindole-2-hexaneperoxoïque (1:1).

7. Composition selon l'une quelconque des revendications précédentes, dans laquelle le dérivé d'acide imido-percarboxylique est l'acide phthalimidopéroxycaproïque.

8. Composition selon l'une quelconque des revendications précédentes, dans laquelle la quantité en matières actives du dérivé d'acide imido-percarboxylique va de 0,01 à 5 % en poids, notamment de 0,01 à 2 % en poids, de préférence de 0,15 à 1,5% en poids et mieux de 0,3 à 1 % en poids, par rapport au poids total de la composition.

9. Composition selon l'une quelconque des revendications précédentes, dans laquelle l'ester d'acide aminé est le N-lauroylsarcosinate d'isopropyle.

10. Composition selon l'une quelconque des revendications précédentes, **caractérisée en ce que** ledit ester d'acide aminé est présent en une teneur allant de 0,05 à 50% en poids, de préférence de 1 à 30% en poids et plus préférentiellement de 1 à 10% en poids par rapport au poids total de la composition.

11. Composition selon l'une quelconque des revendications précédentes, **caractérisée en ce que** l'ester d'acide aminé N-acylé et le dérivé d'acide imido- percarboxylique de formule (I) sont présents dans la composition selon l'invention en des teneurs en matières actives telles que le ratio ester d'acide aminé N-acylé sur dérivé d'acide imido- percarboxylique va de 5 à 25.

12. Procédé de traitement cosmétique consistant à appliquer sur les matières kératiniques une composition selon l'une quelconque des revendications 1 à 11.

13. Procédé de solubilisation d'un dérivé d'acide imido-percarboxylique par un ester d'acide aminé N-acylé de formule (A)
R'₁(CO)N(R'₂)CH(R'₃)(CH₂)ₙ(CO)OR'₄ (A)
dans laquelle :
n est un entier égal à 0,1 ou 2,
R'₁ représente un radical alkyle ou alcényle en C₅ à C₂₁, linéaire ou ramifié,
R'₂ représente un atome d'hydrogène ou un groupe alkyle en C₁ à C₃,
R'₃ représente un radical choisi dans le groupe formé par un atome d'hydrogène, un groupe méthyle, un groupe éthyle, une chaîne alkyle linéaire ou ramifiée en C₃ ou C₄,
R'₄ représente un radical alkyle en C₁ à C₁₀ linéaire ou ramifié, ou un radical alcényle en C₂ à C₁₀ linéaire ou ramifié ou un reste stérol.
